# EUROPÄISCHE PATENTSCHRIFT

(11) **EP 1 436 306 B1**
(45) Veröffentlichungstag und Bekanntmachung des Hinweises auf die Patenterteilung: **28.03.2007**
(21) Anmeldenummer: 02735317.6
(22) Anmeldetag: 27.04.2002
(51) Int. Cl.: C07H 1/00, C07H 15/04, C07H 15/08

(54) **VERFAHREN ZUR HERSTELLUNG VON TENSIDGEMISCHEN**
METHOD FOR THE PRODUCTION OF SURFACE ACTIVE AGENT MIXTURES
PROCEDE DE PRODUCTION DE MELANGES D'AGENTS TENSIOACTIFS

(30) Priorität: 08.05.2001 DE 10122255
(43) Veröffentlichungstag der Anmeldung: 14.07.2004
(73) Patentinhaber: Cognis IP Management GmbH, 40589 Düsseldorf (DE)
(72) Erfinder: BEHLER, Ansgar, 46240 Bottrop (DE); SCHMID, Karl, Heinz, 40822 Mettmann (DE); FOLGE, Almud, 40764 Langenfeld (DE)
(86) Internationale Anmeldenummer: PCT/EP2002/004694
(87) Internationale Veröffentlichungsnummer: WO 2002/090369

(56) Entgegenhaltungen:
- WO-A-97/42299
- WO-A-99/24538
- US-A- 4 599 188
- US-A- 4 806 275
- US-A- 5 179 201
- US-A- 5 908 928

## Beschreibung

### Gebiet der Erfindung

Die Erfindung betrifft ein Verfahren zur Herstellung von Alkyl- und/oder Alkenyloligoglycosid-Ethercarbonsäuren enthaltenden Tensidgemischen durch Umsetzung einer wässrigen Lösung von Alkyl- und/oder Alkenyloligoglycosiden mit ω-Halogencarbonsäuren, deren Salze oder Ester in Gegenwart von Alkali.

### Stand der Technik

Die Herstellung von Alkyl- und/oder Alkenyloligoglycosid-Ethercarbonsäuren erfolgt bekanntlich durch Umsetzung von Alkyl- und/oder Alkenyloligoglycosiden mit Halogencarbonsäuren im alkalischen Medium. Da Alkyl- und/oder Alkenyloligoglycoside im geschmolzenen Zustand sehr hohe Viskositäten aufweisen, ist bei der Umsetzung der Zusatz geeigneter Lösungsmittel erforderlich. Im allgemeinen werden organische, aprotische Lösungsmittel verwendet, so dass eine Hydrolyse der Halogencarbonsäuren bzw. deren Salze oder Ester vermieden wird. In der internationalen Patentanmeldung **WO 97/42299** wird die Umsetzung mit Toluol als Lösungsmittel beschrieben. Hierbei wird zunächst eine wässrige Lösung aus Alkyl- und/oder Alkenyloligoglycosiden mittels Toluol als Schleppmittel entwässert, um eine Hydrolyse des eingesetzten Natriumchloracetats zu verhindern, und anschliessend die Carboxylierung in Toluol durchgeführt.

Die Aufgabe der vorliegenden Erfindung hat nun darin bestanden, ein einfacheres und kostengünstigeres Verfahren zur Herstellung von Alkyl- und/oder Alkenyloligoglycosid-Ethercarbonsäuren zur Verfügung zu stellen, bei dem auf die Entwässerung der Alkyl- und/oder Alkenyloligoglycosid-Lösung verzichtet werden kann und die Umsetzung mit ω-Halogencarbonsäuren, deren Salze oder Ester zu Alkyl- und/oder Alkenyloligoglycosid-Ethercarbonsäuren ohne Zusatz organischer Lösungsmittel in guten Ausbeuten möglich ist.

### Beschreibung der Erfindung

Gegenstand der vorliegenden Erfindung ist ein Verfahren zur Herstellung von Alkyl- und/oder Alkenyloligoglykosid-Ethercarbonsäuren enthaltenden Tensidgemischen, dadurch gekennzeichnet, dass man eine wässrige Lösung aus mindestens einem Alkyl- und/oder Alkenyloligoglycosid in Gegenwart von Alkali mit einer ω-Halogencarbonsäure, deren Salz oder Ester umsetzt.

Überraschenderweise wurde gefunden, dass sich Alkyl- und/oder Alkenyloligoglycosid-Ethercarbonsäuren durch Umsetzung einer wässrigen Lösung von Alkyl- und/oder Alkenyloligoglycosiden mit ω-Halogencarbonsäuren, deren Salzen oder Ester und Alkali in guten Ausbeuten herstellen lassen, ohne dass es einer Entwässerung bedarf. Dieses Verfahren ist im Vergleich zum Stand der Technik nicht nur umweltfreudlicher aufgrund von Vermeidung organischer Lösungsmittel, sondern darüber hinaus ebenfalls kostengünstiger und einfacher in der Durchführung. Besonders vorteilhaft ist, dass die so erhaltenen Tensidgemische direkt in kosmetischen Formulierungen eingesetzt werden können, ohne dass organische Lösungsmittel kostenintensiv entfernt werden müssen.

### Alkyl- und/oder Alkenyloligoglykoside

Zur Herstellung der erfindungsgemässen Tensidgemische werden Alkyl- und Alkenyloligoglykoside der Formel **(I)** eingesetzt,

**R¹O-[G]ₚ** **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in **StarchlStärke 45, 281 (1993), B.Salka in Cosm.Toil. 108, 89 (1993)** sowie J.Kahre et al. in **SÖFW-Journal Heft 8, 598 (1995)** verwiesen.
Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich femer auch von primären Alkoholen mit 12 bis 22, vorzugsweise 12 bis 18 und insbesondere 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Alkylierungsmittel

Zur Herstellung der erfindungsgemässen Tensidgemische werden ω-Halogencarbonsäuren, deren Salze oder Ester eingesetzt. Als ω-Halogencarbonsäuren, deren Salze oder Ester kommen erfindungsgemäss alle derartigen aus der Literatur bekannten Verbindungen in Frage. Vorzugsweise wird Kalium- oder Natriummonochloracetat (MCA) eingesetzt.

### Verfahren

Zur Herstellung der erfindungsgemässen Tensidgemische wird eine wässrige Lösung aus Alkyl- und/oder Alkenyloligoglycosiden, vorzugsweise C_{12/14} -Alkylpolyglucoside, in einem Rührbehälter vorgelegt. Diese wässrige Lösung enthält höchstens 70, vorzugsweise 20 bis 65 und insbesondere 50 bis 60 Gew.-% Alkyl- und/oder Alkenyloligoglycoside - bezogen auf den Aktivsubstanzgehalt. Unter Stickstoffatmosphäre wird die Lösung auf 50 bis 100, vorzugsweise 60 bis 85 und insbesondere 80 °C erhitzt und anschließend Alkali zugegeben. Unter Alkali werden im Sinne der Erfindung feste Alkalihydroxide oder Alkalicarbonate oder in Wasser gelöstes Alkalihydroxid, vorzugsweise Natriumhydroxid oder Alkalicarbonat verstanden. Vorzugsweise wird festes Alkalihydroxid, insbesondere Natriumhydroxid, wie beispielsweise NaOH Prills eingesetzt.

Nach 1 bis 4, vorzugsweise 1 Stunde Rührzeit (leichter Temperaturanstieg bis zu 90 °C) wird die ω-Halogencarbonsäure, deren Salz oder Ester, vorzugsweise Natriumchloracetat (MCA), zugegeben und solange bei 50 bis 100, vorzugsweise 80 °C gerührt, bis eine vollständige Umsetzung der eingesetzten ω-Halogencarbonsäure erfolgt ist. Die Reaktionskontrolle erfolgt dabei über die freigewordene Menge Alkalihalogenid, vorzugsweise Natriumchlorid. In einer besonderen Ausführungsform der Erfindung setzt man das Alkyl- und/oder Alkenyloligoglycosid mit der ω-Halogencarbonsäure, deren Salz oder Ester, vorzugsweise Natriummonochloracetat (MCA), im Molverhältnis 1 : 0,5 bis 1 : 3,5 und vorzugsweise 1 : 1 bis 1 : 2 um. In einer weiteren Ausführungsform der Erfindung wählt man ein Molverhältnis Alkali : ω-Halogencarbonsäure, deren Salz oder Ester von 1 : 0,5 bis 1: 1,5 und vorzugsweise 1:1 1 bis 1 :1,3. Die Umsetzung erfolgt ohne Zugabe organischer Lösungsmittel.
Nach einer Reaktionszeit von 2 bis 8, vorzugsweise 3 bis 6 und insbesondere 3 bis 5 Stunden erhält man eine Ausbeute von 30 bis 100 % Alkyl- und/oder Alkenyloligoglycosid-Ethercarbonsäuren bezogen auf die eingesetzte Menge Alkyl- und/oder Alkenyloligoglycoside.

Die erfindungsgemäß hergestellten Tensidgemische enthalten vorzugsweise 50 bis 70 Gew. %, insbesondere 10 bis 45 Gew. % an Alkyl- und/oder Alkenyloligoglykosid- Ethercarbonsäuren sowie 0 bis 50 Gew. %, insbesondere 5 bis 30 Gew. % an unumgesetzten Alkyl- und/oder Alkenyloligoglykosid.
Der zu 100 Gew. % fehlende Rest des Tensidgemisches wird vorzugsweise aus sich bildendem Alkalihalogenid und aus der Halogencarbonsäure freiwerdender Carbonsäure sowie Wasser gebildet.

Falls gewünscht, kann in einem anschließenden Verfahrensschritt durch herkömmliche Trocknungsverfahren, bevorzugt Gefriertrocknung, Wasser entzogen werden, so dass man ein trockenes Pulver des erfindungsgemäß hergestellten Tensidgemisches mit einem Restwassergehalt von maximal 5 Gew. %, bevorzugt maximal 3 Gew. % und besonders bevorzugt maximal 1 Gew. % - bezogen auf das getrocknete Tensidgemisch - erhält.

### Gewerbliche Anwendbarkeit

Die erfindungsgemässen Tensidgemische können durch Zusatz von Wasser auf beliebige Konzentrationen eingestellt werden, wobei der Wassergehalt 20 bis 90, vorzugsweise 30 bis 80 und insbesondere 40 bis 70 Gew.-% betragen kann.

Das erfindungsgemässe Tensidgemisch kann in oberflächenaktiven Zubereitungen, wie beispielsweise Wasch- und Spülmittel, Haushaltsreiniger sowie kosmetische und/oder pharmazeutische Zubereitungen eingesetzt werden. Diese oberflächenaktiven Zubereitungen können als weitere Hilfs- und Zusatzstoffe Perlglanzwachse, Konsistenzgeber, Verdickungsmittel, Überfettungsmittel, Stabilisatoren, Siliconverbindungen, Fette, Wachse, Lecithine, Phospholipide, Antioxidantien, Deodorantien, Antitranspirantien, Antischuppenmittel, Quellmittel, Tyrosininhibitoren, Hydrotrope, Solubilisatoren, Konservierungsmittel, Parfümöle, Farbstoffe, weitere Tenside und dergleichen enthalten. Als kosmetische und/oder pharmazeutische Zubereitungen kommen beispielsweise Mund- und Zahnpflegemittel, Haarshampoos, Haarlotionen, Schaumbäder, Duschbäder, Cremes, Gele, Lotionen, alkoholische und wässrig/alkoholische Lösungen und Emulsionen in Frage.

### Beispiele

### Beispiel 1: Herstellung der Tensidmischung 1

24,41 kg (0,029 kmol) Plantacare 1200 UP (C_{12/14}-Alkylpolyglucosid), enthaltend 50 Gew.-% C_{12/14}-Alkylpolyglucosid, werden in einem Rührbehälter vorgelegt und unter Stickstoffatmosphäre auf 80 °C erhitzt. Nach Erreichen der Temperatur werden 1,51 kg (0,0377 kmol) NaOH Prills zugegeben und eine Stunde nachgerührt, wobei es zu einem leichten Temperaturanstieg auf 90 °C kommt. Anschliessend werden 4,39 kg (0,0377 kmol) Natriumchloracetat (MCA) zugegeben und solange bei 80 °C gerührt, bis eine vollständige Umsetzung der eingesetzten MCA erfolgt ist. Die Reaktionskontrolle erfolgt dabei über die freigewordene Menge NaCl. Nach einer Reaktionszeit von 4,5 Stunden war die Reaktion abgeschlossen. Es entstand eine viskose, hellgelbe Flüssigkeit mit der folgenden Zusammensetzung:

| | |
|---|---|
| **freies, nicht umgesetztes APG:** | **15,3 %** |
| **NaCl:** | **4,3 %** |
| **Glykolsäure:** | **2,8 %** |
| **Trockenrückstand:** | **51,7 %** |
| **C_{12/14}-Alkylpolyglucosidethylcarboxylat:** | **29,3 %** |

### Beispiel 2: Herstellung der Tensidmischung 2:

673,3 g (0,8 mol) Plantacare 1200 UP (C_{12/14} Alkylpolyglucosid), enthaltend 50 Gew.-% C_{12/14}- Alkylpolyglucosid, werden in einem Rührbehälter vorgelegt und unter Stickstoffatmosphäre auf 80 °C erhitzt. Nach Erreichen der Temperatur werden 96,0 kg (2,4 mol) NaOH Prills zugegeben und eine Stunde nachgerührt, wobei es zu einem leichten Temperaturanstieg auf 105°C kommt. Anschliessend werden 279,6 g (2,4 mol) Natriumchloracetat (MCA) zugegeben und solange bei 90 °C gerührt, bis eine vollständige Umsetzung der eingesetzten MCA erfolgt ist. Die Reaktionskontrolle erfolgt dabei über die freigewordene Menge NaCl. Nach einer Reaktionszeit von 3 Stunden war die Reaktion abgeschlossen. Es entstand eine viskose, hellgelbe Flüssigkeit mit der folgenden Zusammensetzung:

| | |
|---|---|
| freies, nicht umgesetztes APG: | 15,8 % |
| NaCl: | 13,5 % |
| Glykolsäure: | 5,7 % |
| Trockenrückstand: | 67,9 % |
| C_{12/14} Alkylpolyglucosidmethylcarboxylat: | 28,9% |

## Patentansprüche

1. Verfahren zur Herstellung von Alkyl- und/oder Alkenyloligoglykosid-Ethercarbonsäuren enthaltenden Tensidgemischen, **dadurch gekennzeichnet, dass** man eine wässrige Lösung aus mindestens einem Alkyl- und/oder Alkenyloligoglycosid in Gegenwart von Alkali mit einer ω-Halogencarbonsäure, deren Salz oder Ester umsetzt.

2. Verfahren nach Anspruch 1, **dadurch gekennzeichnet, dass** man Alkyl- und Alkenyloligoglykoside der Formel **(I)** einsetzt,
**R¹O-[G]ₚ** **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Verfahren nach den Ansprüchen 1 und/oder 2, **dadurch gekennzeichnet, dass** man als ω-Halogencarbonsäure, deren Salz oder Ester Natriummonochloracetat einsetzt.

4. Verfahren nach mindestens einem der Ansprüche 1 bis 3, **dadurch gekennzeichnet, dass** man das Alkyl- und/oder Alkenyloligoglycosid mit einer ω-Halogencarbonsäure, deren Salz oder Ester im Molverhältnis 1 : 0,5 bis 1:3,5 umsetzt.

5. Verfahren nach mindestens einem der Ansprüche 1 bis 4, **dadurch gekennzeichnet, dass** man Alkyl- und/oder Alkenyloligoglycoside mit einer ω-Halogencarbonsäure, deren Salz oder Ester im Molverhältnis 1:1 1 bis 1 : 2 umsetzt.

6. Verfahren nach mindestens einem der Ansprüche 1 bis 5, **dadurch gekennzeichnet, dass** man ein Molverhältnis Alkali : ω-Halogencarbonsäure, deren Salz oder Ester von 1 : 0,5 bis 1 : 1,5 wählt.

7. Verfahren nach mindestens einem der Ansprüche 1 bis 6, **dadurch gekennzeichnet, dass** man ein Molverhältnis Alkali : einer ω-Halogencarbonsäure, deren Salz oder Ester 1 : 1 bis 1 : 1,3 wählt.

8. Verfahren nach mindestens einem der Ansprüche 1 bis 7, **dadurch gekennzeichnet, dass** man die Umsetzung bei einer Temperatur von 50 bis 100 °C durchführt.

9. Verfahren nach mindestens einem der Ansprüche 1 bis 8, **dadurch gekennzeichnet, dass** man keine weiteren Lösungsmittel zusetzt.

10. Verfahren nach mindestens einem der Ansprüche 1 bis 9, **dadurch gekennzeichnet, dass** die wässrige Lösung höchstens 70 Gew.-% Alkyl- und/oder Alkenyloligoglycoside - bezogen auf den Aktivsubstanzgehalt - enthält.

11. Verfahren nach mindestens einem der Ansprüche 1 bis 10, **dadurch gekennzeichnet, dass** man das Umsetzungsprodukt der wässrigen Lösung aus mindestens einem Alkyl- und/oder Alkenyloligoglycosid in Gegenwart von Alkali mit einer ω-Halogencarbonsäure, deren Salz oder Ester nachfolgend bis auf einen Restwassergehalt von maximal 5 Gew.% trocknet.

## Claims

1. A process for the production of surfactant mixtures containing alkyl and/or alkenyl oligoglycoside ether carboxylic acids, **characterized in that** an aqueous solution of at least one alkyl and/or alkenyl oligoglycoside is reacted with a ω-halocarboxylic acid, salt or ester in the presence of alkali.

2. A process as claimed in claim 1, **characterized in that** alkyl and alkenyl oligoglycosides corresponding to formula **(I)**:
**R¹O[G]ₚ** **(I)**
in which R¹ is an alkyl and/or alkenyl group containing 4 to 22 carbon atoms, G is a sugar unit containing 5. or 6 carbon atoms and p is a number of 1 to 10, are used.

3. A process as claimed in claims 1 and/or 2, **characterized in that** sodium monochloroacetate is used as the ω-halocarboxylic acid, salt or ester.

4. A process as claimed in at least one of claims 1 to 3, **characterized in that** the alkyl and/or alkenyl oligoglycoside is reacted with a ω-halocarboxylic, salt or ester thereof in a molar ratio of 1:0.5 to 1:3.5.

5. A process as claimed in at least one of claims 1 to 4, **characterized in that** alkyl and/or alkenyl oligoglycosides are reacted with a *ω-*halocarboxylic acid, salt or ester thereof in a molar ratio of 1:1 to 1:2.

6. A process as claimed in at least one of claims 1 to 5, **characterized in that** a molar ratio of alkali to ω-halocarboxylic acid, salt or ester of 1:0.5 to 1:1.5 is selected.

7. A process as claimed in any of claims 1 to 6, **characterized in that** a molar ratio of alkali to ω-halocarboxylic acid, salt or ester of 1:1 to 1:1.3 is selected.

8. A process as claimed in at least one of claims 1 to 7, **characterized in that** the reaction is carried out at a temperature of 50 to 100°C.

9. A process as claimed in at least one of claims 1 to 8, **characterized in that** no other solvents are added.

10. A process as claimed in at least one of claims 1 to 9, **characterized in that** the aqueous solution contains at most 70% by weight of alkyl and/or alkenyl oligoglycosides, based on the active substance content.

11. A process as claimed in at least one of claims 1 to 10, **characterized in that** the product of the reaction of the aqueous solution of at least one alkyl and/or alkenyl oligoglycoside with a ω-halocarboxylic acid, salt or ester thereof in the presence of alkali is dried to a residual water content of at most 5% by weight.

## Revendications

1. Procédé de préparation de mélanges d'agents tensioactifs contenant des acides éthercarboxyliques d'oligoglycosides d'alkyle et/ou d'alcényle,
**caractérisé en ce qu'**
on fait réagir une solution aqueuse d'au moins un oligoglycoside d'alkyle et/ou d'alcényle en présence de composés alcalins avec un acide carboxylique ω-halogéné, son sel ou ester.

2. Procédé selon la revendication 1,
**caractérisé en ce qu'**
on utilise des oligoglycosides d'alkyle et d'alcényle de formule (I)
R¹O-[G]ₚ (I)
dans laquelle R¹ représente un radical alkyle et/ou alcényle comportant de 4 à 22 atomes de carbone, G un radical sucre comportant de 5 à 6 atomes de carbone et p des nombres de 1 à 10.

3. Procédé selon les revendications 1 et/ou 2,
**caractérisé en ce qu'**
on utilise du monochloroacétate de sodium en tant qu'acide carboxylique ω-halogéné, son sel ou ester.

4. Procédé selon au moins l'une des revendications 1 à 3,
**caractérisé en ce qu'**
on fait réagir l'oligoglycoside d'alkyle et/ou d'alcényle avec un acide carboxylique ω-halogéné, son sel ou ester dans un rapport molaire de 1:0,5 à 1:3,5.

5. Procédé selon au moins l'une des revendications 1 à 4,
**caractérisé en ce qu'**
on fait réagir des oligoglycosides d'alkyle et/ou d'alcényle avec un acide carboxylique ω-halogéné, son sel ou ester dans un rapport molaire de 1:1 à 1:2.

6. Procédé selon au moins l'une des revendications 1 à 5,
**caractérisé en ce qu'**
on choisit un rapport molaire composés alcalins:acide carboxylique ω-halogéné, son sel ou ester de 1:0,5 à 1:1,5.

7. Procédé selon au moins l'une des revendications 1 à 6,
**caractérisé en ce qu'**
on choisit un rapport molaire composés alcalins:acide carboxylique ω-halogéné, son sel ou ester de 1:1 à 1:1,3.

8. Procédé selon au moins l'une des revendications 1 à 7,
**caractérisé en ce qu'**
on met en oeuvre la réaction à une température de 50 à 100 °C.

9. Procédé selon au moins l'une des revendications 1 à 8,
**caractérisé en ce qu'**
on n'ajoute pas d'autre solvant.

10. Procédé selon au moins l'une des revendications 1 à 9,
**caractérisé en ce que**
la solution aqueuse contient au plus 70 % en poids d'oligoglycosides d'alkyle et/ou d'alcényle par rapport à la teneur en substance active.

11. Procédé selon au moins l'une des revendications 1 à 10,
**caractérisé en ce qu'**
on sèche ensuite le produit de la réaction de la solution aqueuse d'au moins un oligoglycoside d'alkyle et/ou d'alcényle, en présence de composés alcalins avec un acide carboxylique ω-halogéné, son sel ou ester jusqu'à une teneur résiduelle en eau de 5 % en poids au maximum.
